(19) Europäisches Patentamt
European Patent Office
Office européen des brevets

(11) Veröffentlichungsnummer: **0 034 731**
**B1**

(12) **EUROPÄISCHE PATENTSCHRIFT**

(45) Veröffentlichungstag der Patentschrift: **30.10.85**

(21) Anmeldenummer: **81100746.7**

(22) Anmeldetag: **03.02.81**

(51) Int. Cl.$^4$: **C 07 C 69/743,** C 07 C 33/46, C 07 C 67/14, C 07 C 67/10, C 07 C 25/13, C 07 C 29/136, A 01 N 53/00

(54) **Trifluormethylbenzylester, ihre Zwischenprodukte, Verfahren zu ihrer Herstellung und deren Verwendung in Schädlingsbekämpfungsmitteln.**

(30) Priorität: **15.02.80 DE 3005722**

(43) Veröffentlichungstag der Anmeldung: **02.09.81 Patentblatt 81/35**

(45) Bekanntmachung des Hinweises auf die Patenterteilung: **30.10.85 Patentblatt 85/44**

(84) Benannte Vertragsstaaten: **BE CH DE FR GB IT LI NL**

(56) Entgegenhaltungen:
**EP-A-0 016 513**
**DE-A-2 333 849**
**DE-A-2 757 406**
**FR-A-1 560 091**
**FR-A-2 147 310**
**US-A-3 465 051**
**US-A-4 063 022**
**US-A-4 132 737**

**J. Amer. Chem. Soc. 62 (1940), S. 1839,1840**

**Die Akte enthält technische Angaben, die nach dem Eingang der Anmeldung eingereicht wurden und die nicht in dieser Patentschrift enthalten sind.**

(73) Patentinhaber: **BAYER AG**
**Konzernverwaltung RP Patentabteilung**
**D-5090 Leverkusen 1 Bayerwerk (DE)**

(72) Erfinder: **Marhold, Albrecht, Dr.**
**Carl-Duisbergstrasse 329**
**D-5090 Leverkusen (DE)**
Erfinder: **Naumann, Klaus, Dr.**
**Richard-Wagner-Strasse 9**
**D-5090 Leverkusen (DE)**
Erfinder: **Fuchs, Rainer, Dr.**
**Roeberstrasse 8**
**D-5600 Wuppertal 1 (DE)**
Erfinder: **Hammann, Ingeborg, Dr.**
**Belfortstrasse 9**
**D-5000 Köln (DE)**
Erfinder: **Behrenz, Wolfgang, Dr.**
**Untergründemich 14**
**D-5063 Overath (DE)**
Erfinder: **Homeyer, Bernhard, Dr.**
**Obere Strasse 28**
**D-5090 Leverkusen 3 (DE)**
Erfinder: **Stendel, Wilhelm, Dr.**
**In den Birken 55**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 034 731 B1

**Beschreibung**

Die Erfindung betrifft neue halogensubstituierte Trifluormethylbenzylester, ein Verfahren, zu deren Herstellung und deren Verwendung in Schädlingsbekämpfungsmitteln, insbesondere in Insektiziden und Akariziden.

Es ist bekannt, daß bestimmte Fluorbenzylester, wie z.B. 3-Cyclopropylidenmethyl- und 3-Cyclobutylidenmethyl-2,2-dimethyl-cyclopropan-1-carbonsäure-pentafluorbenzylester, insektizide und akarizide Eigenschaften aufweisen (vergleiche DE—OS 2 810 634).

Die Wirkung dieser Verbindungen ist jedoch, insbesondere bei niedrigen Wirkstoffkonzentrationen und Aufwandmengen, nicht immer zufriedenstellend.

Aus der französischen Offenlegungsschrift 2 147 310 ist Chrysanthemumsäure-p-trifluormethylbenzylester bekannt. Diese Verbindung besitzt wie alle Chrysanthemumsäureester den Nachteil einer gewissen Lichtinstabilität. Außerdem wiesen p-Trifluormethylbenzylester von bei Pyrethroiden üblichen Säuren bei niedrigen Aufwandkonzentrationen nur unbefriedigende Wirkungen auf.

Am DE—OS 2333 849, 2 757 406, US—PS 3 465 051, 4,132,737, 4 063 022, FR—OS 1 560 091 sind halogensubstituierte Trifluormethylbenzylalkohole und ihre Derivate bekannt. Es geht jedoch nichts darüber hervor, daß Trifluormethyl-tetrahalogenbenzylalkohole zu hervorragend wirksamen Pyrethroiden führen.

Aus der älteren, nicht vorveröffentlichten Europäischen Anmeldung 16 513 ist Permethrinsäure-4-trifluormethyltetrafluorbenzylester bekannt.

Gegenstand der vorliegenden Erfindung sind

(1) neue halogensubstituierte Trifluormethylbenzylester der Formel I

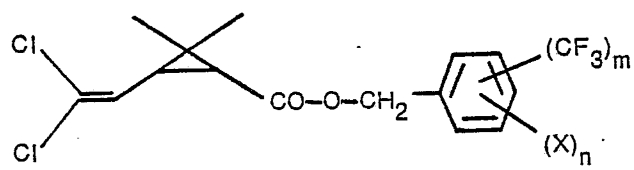

(I)

in welcher

X für Halogen steht,

m für 1, 2 oder 3 steht und

n für 0, 1, 2, 3 oder 4 steht,

mit der Maßgabe, daß die Summe von m und n zwischen 2 und 5 liegt, ausgenommen der 4-Trifluormethyl-tetrafluorbenzylester,

(2) eine Verfahren zur Herstellung der neuen Verbindungen der Formel (I), dadurch gekennzeichnet, daß man die Permethrinsäure der Formel II

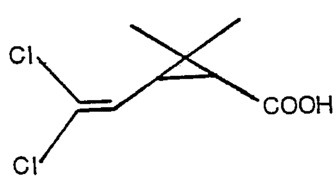

(II)

oder reaktionsfähige Derivate derselben mit Trifluormethylbenzylalkoholen der Formel III

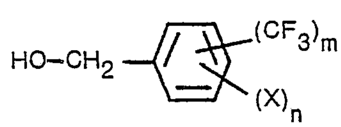

(III)

in welcher

X, m und n die oben angegebenen Bedeutungen haben, mit Ausnahme von 4-Trifluormethyltetrafluorbenzylalkohol

oder mit reaktionsfähigen Derivaten derselben gegebenenfalls in Gegenwart von Säureakzeptoren und gegebenenfalls in Gegenwart von Verdünnungsmitteln umsetzt;

(3) neue Trifluormethylbenzylalkohole und deren reaktionsfähige Derivate der Formel III a

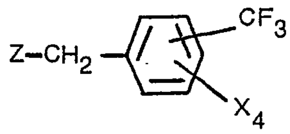

(III–a)

in welcher
Z für Hydroxy, Chlor oder Brom steht,
X für Halogen steht,
mit Ausnahme des 4-Trifluormethylbenzylalkohols oder seiner reaktionsfähigen Derivate

(4) Verfahren zur Herstellung der neuen Verbindungen der Formel (IIIa), dadurch gekennzeichnet, daß man

(a) zur Herstellung von Verbindungen der Formel (IIIa), in welcher Z für Hydroxy steht, Trifluormethylbenzol-carbonylverbindungen der Formel IV

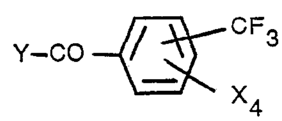

(IV)

in welcher
X die oben angegebenen Bedeutungen hat und
Y für Wasserstoff, Fluor oder Chlor steht, ausgenommen eine verbindung der Formel

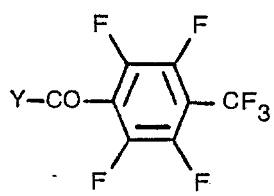

mit Natriumboranat, (Natriumtetrahydridoborat) oder Lithiumalanat (Lithiumtetrahydridoaluminat) gegebenenfalls in Gegenwart eines Verdünnungsmittels umsetzt oder

(b) zur Herstellung von Verbindungen der Formel (IIIa), in welcher Z für Chlor oder Brom steht, Trifluormethyltoluolderivate der Formel V

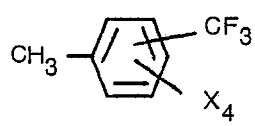

(V)

in welcher
X die oben angegebenen Bedeutungen hat, ausgenommen die Verbindung 4-Trifluormethyltetrafluortoluol
mit Chlor oder Brom, gegebenenfalls in Gegenwart eines Verdünnungsmittels und gegebenenfalls in Gegenwart eines Katalysators umsetzt.

Die neuen Trifluormethylbenzylester der Formel (I) zeichnen sich durch hohe pestizide, insbesondere insektizide und akarizide Wirksamkeit aus.

Überraschenderweise ziegen die erfindungsgemäßen Verbindungen der Formel (I) eine erheblich höhere insektizide und akarizide Wirkung als aus dem Stand der Technik bekannte Verbindungen analoger Konstitution und gleicher Wirkungsrichtung.

In Formel (I) stehen vorzugsweise
X für Fluor oder Chlor,
m für 1 oder 2 und
n für 3 oder 4

Verwendet man als Ausgangsstoffe bei Verfahren (2) 3-Trifluormethyl-2,4,5,6-tetrafluorbenzylchlorid,

so kann die Reaktion dieser Verbindung durch folgendes Formelschema skizziert werden:

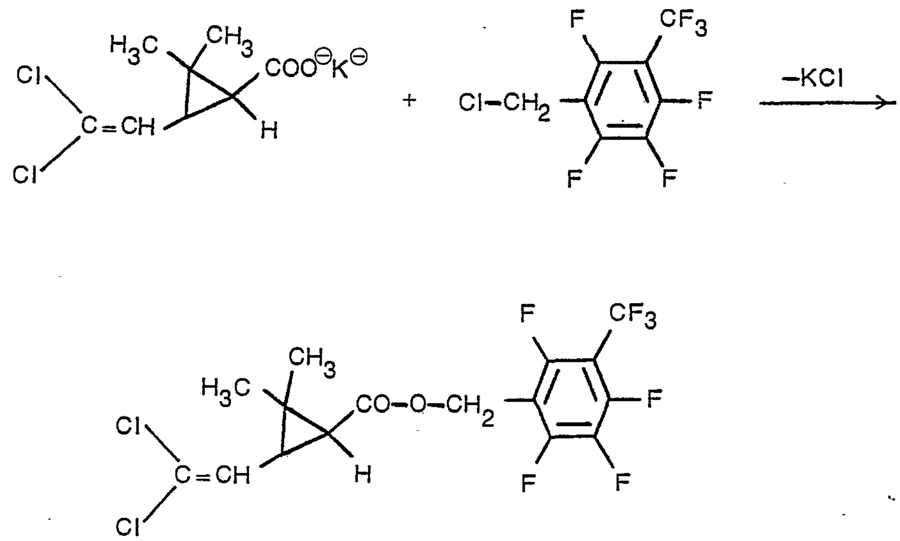


Die weiter als Ausgangsstoffe zu verwendenden Trifluormethylbenzylalkohole bzw. ihre reaktionsfähigen Derivate sind durch Formel (III) bzw. (IIIa) definiert. Dabei hat X die gleiche Bedeutung, wie sie oben bei der Definition der Verbindungen der Formel (I) angegeben ist und

Z steht vorzugsweise für Hydroxy oder Chlor.

Als Beispiele seien genannt:

3-Trifluormethyl-2,4,5,6-tetrafluor-benzylalkohol,
2-Trifluormethyl-3,4,5,6-tetrafluor-benzylalkohol,
4-Trifluormethyl-2,3,5,6-tetrachlor-benzylchlorid,
3-Trifluormethyl-2,4,5,6-tetrachlor-benzylchlorid und
2-Trifluormethyl-3,4,5,6-tetrachlor-benzylchlorid.

Man erhält die neuen Trifluormethylbenzylalkohole der Formel (IIIa), wie unter (4a) dargelegt, indem man Trifluormethylbenzol-carbonylverbindungen der Formel IV (oben), wie z.B. 2- oder 3-Trifluormethyl-tetrafluor-benzoyl-fluorid oder -chlorid oder 2- oder 3-Trifluormethyl-tetrafluorbenzaldehyd, beispielsweise mit Natriumboranat gegebenenfalls in Gegenwart eines Verdünnungsmittels, wie z.B. Isopropanol, Tetrahydrofuran oder Dioxan, bei Temperaturen zwischen 10 und 100°C umsetzt. Das Reaktionsgemisch wird nach Reaktionsende mit Eiswasser verdünnt und angesäuert. Dann wird mit einem mit Wasser nicht mischbaren Lösungsmittel, wie z.B. Methylenchlorid extrahiert, der Extrakt getrocknet, filtriert und destilliert.

Die neuen Trifluormethylbenzylhalogenide der Formel (IIIa) (Chloride bzw. Bromide) erhält man, wie unter (4b) dargelegt, indem man Trifluormethyl-toluolderivate der Formel V (oben), wie z.B. 4-Trifluormethyl-3-chlor-tuluol, mit Chlor bzw. Brom. gegebenenfalls in Gegenwart eines Katalysators, wie z.B. Eisen(II)-sulfid, und vorzugsweise ohne Verwendung eines Verdünungsmittels bei Temperaturen zwischen 20 und 150°C, vorzugsweise zwischen 50 und 120°C, umsetzt und die Produkte durch Destillation isoliert.

Das unter (2) dargelegte Herstellungsverfahren wird gegebenenfalls unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methyl-ethyl-, Methyl-isopropyl- und Methyl-isobutylketon, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Diazabizycloundecen, Tetramethylethylendiamin und Pyridin.

Die Reaktionstemperatur kann innerhalb eines größeren Bereichs variiert werden. Im allgemeinen arbeitet man zwischen 0 und 150°C, vorzugsweise bei 20 bis 100°C.

Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Bei der Verfahrensvariante (2a) werden in einer bevorzugten Ausführungsform die Komponenten bis zum Ende der Gasentwicklung erhitzt und das Produkt wird dann durch Hochvakuumdestillation isoliert.

Die Verfahrensvariante (2b) wird wie in Synthesis *1975*, Seite 805 beschrieben, durchgeführt. Zur Aufarbeitung wird eingeengt und mit verdünnter Salzsäure und Diethylether geschüttelt. Die Etherphase wird eingeengt und der Rückstand wird zur Isolierung des Produktes im Hochvakuum destilliert.

Herstellungsbeispiele

(1) 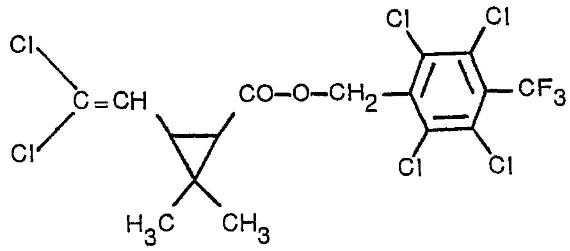

0,1 Mol Kaliumsalz der 3-(2,2-Dichlor-vinyl)-2,2-dimethyl-cyclopropan-1-carbonsäure und 0,1 Mol 4-Trifluormethyl-2,3,5,6-tetrachlor-benzylchlorid werden in 100 ml Acetonitril nach Zugabe von 5 ml Tetramethylethylendiamin, 15 Stunden auf 70°C erhitzt. Das Reaktionsgemisch wird eingeengt und mit verdünnter Salzsäure und Diethylether geschüttelt. Die Etherphase wird eingeengt und der Rückstand am Kugelrohr destiliert. Siedepunkt: 200°C/0,15 mbar.

Analog den vorstehend beschriebenen Herstellungsvarianten können die nachstehend aufgeführten Verbindungen hergestellt werden:

(2) 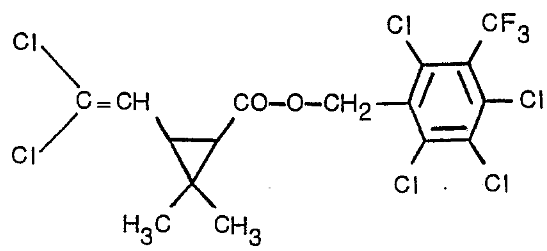

Siedepunkt: 200°C/0,15 mbar

(3) 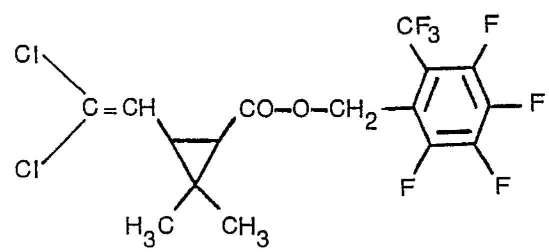

Siedepunkt: 160°C/0,15 mbar.

Beispiele zur Herstellung von Ausgangsverbindungen:

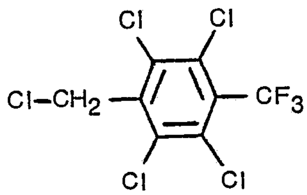

350 g 2-Chlor-4-methyl-benzo-trifluorid werden mit 3 g Eisen(II)sulfid bei 50°C vorgelegt und Chlor eingeleitet. Mit nachlassender Chloraufnahme wird die Temperatur stufenweise auf 120°C gesteigert. Bei einem Gewicht des Reaktionsproduktes von 520 g wird destilliert. Man erhält 250 g 4-Trifluormethyl-2,3,5,6-tetrachlor-benzylchlorid vom Siedepunkt 110—112°C/0,15 mbar.

Analog erhält man

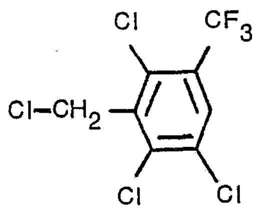

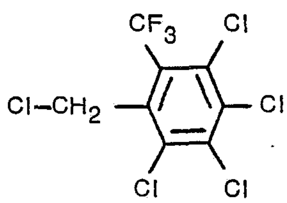

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp, Dysdercus intermedius, Piesma quadrata, Cimex lectularits, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum

padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotaisa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegenbenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Wiese.

Bei der Anwendung gegen Hygiene- und Vorratsschädlinge zwiechnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bakämpfung von Ekto- und Endoparasiten auf dem veterinärmemedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

## Beispiel A

Myzus-Test
Lösungsmittel: 3 Gewichtsteile Aceton
Emulgator: 1 Gewichtseil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1,2.

## Beispiel B

Tetranychus-Test (resistent)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test ziegen z.B. die folgenden Verbindungen der Herstellungsbeispiele überlegene Wirksamkeit gegenüber dem Stand der Technik: 2.

## Beispiel C

Grenzkonzentrations-Test/Bodeninsekten
Testinsekt: Phorbia antiqua-Maden (im Boden)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch

genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2.

Beispiel D

Grenzkonzentrations-Test/Bodeninsekten
Testinsekt: Agrotis segetum-Larven (im Boden)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2.

Beispiel E

$LD_{100}$-Test
Testtiere: Blatta orientalis
Zahl der Testtiere: 10
Lösungsmittel: Aceton

2 Gewichtsteile Wirkstoffe werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100%, daß alle Testtiere abgetötet wurden; 0% bedeutet, daß keine Testtiere abgetötet wurden.

Bei diesem Test zeigen z.B. die folgenden Verbindungen der Herstellungsbeispiele überlegene Wirkung gegenüber dem Stand der Technik: 3.

Beispiel

Test mit Lucilia cuprina res.-Larven
Emulgator: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test ziegen z.B. die folgenden Verbindungen der Herstellungsbeispiele überlegene Wirkung gegenüber dem Stand der Technik: 2.

Beispiel

Test mit Musca autumnalis
Lösungsmittel: 35 Gewichtsteile Äthylenglykolmonomethyläther
35 Gewichtsteile Nonylphenolpolylgkyoläther

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene

Konzentrat mit Wasser auf die jeweils gewünschte Konzentration.

10 adulte Musca autumnalis werden in Petrischalen gebracht, die Filterpapierschieben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigen z.B. die folgenden Verbindungen der Herstellungsbeispiele überlegene Wirkung gegenüber dem Stand der Technik: 3.

**Patentansprüche**

1. Trifluormethylbenzylester der Formel I

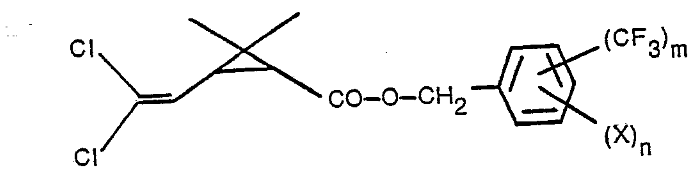

(I)

in welcher

X für Halogen steht,

m für 1, 2 oder 3 steht und

n für 0, 1, 2, 3 oder 4 steht,

mit der Maßgabe, daß die Summe von m und n zwischen 2 und 5 liegt, ausgenommen der 4-Trifluormethyl-tetrafluor-benzylester.

2. Verfahren zur Herstellung der neuen Verbindungen der Formel (I), dadurch gekennzeichnet, daß man die Carbonsäure der Formel II

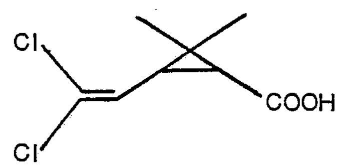

(II)

oder reaktionsfähige Derivate derselben mit Trifluormethylbenzylalkoholen der Formel III

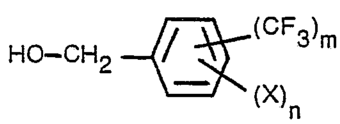

(III)

in welcher

X, m und n die oben angegebenen Bedeutungen haben, mit Ausnahme von 4-Trifluormethyltetrafluorbenzylalkohol oder mit reaktionsfähigen Derivaten derselben gegebenenfalls in Gegenwart von Säureakzeptoren und gegebenenfalls in Gegenwart von Verdünnungsmitteln umsetzt.

3. Trifluormethylbenzylalkohole und deren reaktionsfähige Derivate der Formel IIIa

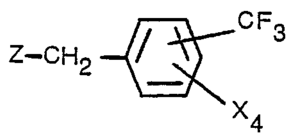

(III–a)

in welcher

Z für Hydroxy, Chlor oder Brom steht,

X für Halogen steht,

mit Ausnahme des 4-Trifluormethyl-tetrafluorbenzylalkohols oder seiner reaktionsfähigen Derivate

4. Verfahren zur Herstellung der neuen Verbindungen der Formel (IIIa), dadurch gekennzeichnet, daß man

(a) zur Herstellung von Verbindungen der Formel (IIIa), in welcher Z für Hydroxy steht, Trifluormethylbenzol-carbonylverbindungen der Formel IV

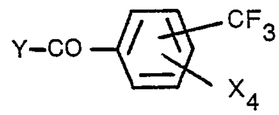

(IV)

in welcher

X die oben angegebenen Bedeutungen hat und

Y für Wasserstoff, Fluor oder Chlor steht, ausgenommen eine verbindung der Formel

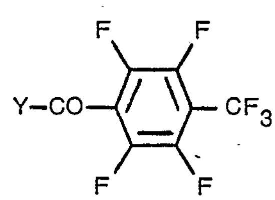

mit Natriumboranat, (Natriumtetrahydridoborat) oder Lithiumalanat (Lithiumtetrahydridoaluminat) gegebenenfalls in Gegenwart eines Verdünnungsmittels umsetzt oder

(b) zur Herstellung von Verbindungen der Formel IIIa, in welcher Z für Chlor oder Brom steht, Trifluormethyltoluolderivate der Formel V

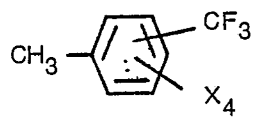

(V)

in welcher

X die oben angegebenen Bedeutungen hat, ausgenommen die Verbindung 4-Trifluormethyltetrafluortoluol mit Chlor oder Brom, gegebenenfalls in Gegenwart eines Verdünnungsmittels und gegebenenfalls in Gegenwart eines Katalysators umsetzt.

5. Schädlingsbekämpfungsmittel, gekennzeichnet durch einen Gehalt an mindestens einem Trifluormethylbenzylester der Formel I.

6. Verwendung von Trifluormethylbenzylestern der Formel (I) zur Bekämpfung von Schädlingen.

7. Verfahren zur Bekämpfung von Schädlingen, dadurch gekennzeichnet, daß man Trifluormethylbenzylester der Formel (I) auf Schädlinge und/oder ihren Lebensraum einwirken läßt.

8. Verfahren zur Herstellung von Schädlingsbekämpfungsmitteln, dadurch gekennzeichnet, daß man Trifluormethylbenzylester der Formel (I) mit Streckmitteln und/oder oberflächenaktiven Mitteln vermischt.

**Claims**

1. Trifluoromethylbenzyl esters of the formula I

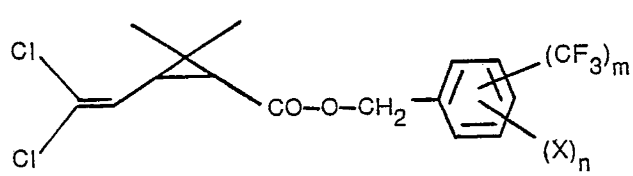

(I)

in which X represents halogen,

m represents 1, 2 or 3 and

n represents 0, 1, 2, 3 or 4,

with the proviso that the sum of m and n is between 2 and 5, with the exception of 4-trifluoromethyl-tetrafluorobenzyl ester.

2. Process for the preparation of the new compounds of the formula (I), characterised in that the carboxylic acid of the formula II

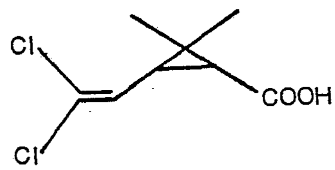

(II)

or reactive derivatives thereof, is reacted with trifluoromethylbenzyl alcohols of the formula III

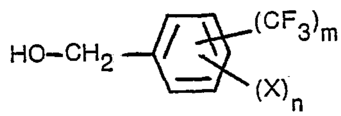

(III)

in which

X, m and n have the abovementioned meanings,

with the exception of 4-trifluoromethyl-tetrafluorobenzyl alcohol, or with reactive derivatives thereof, if appropriate in the presence of acid acceptors and if appropriate in the presence of diluents.

3. Trifluoromethylbenzyl alcohols and reactive derivatives thereof of the formula IIIa

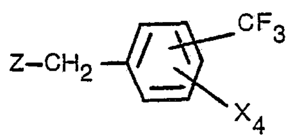

(III—a)

in which

Z represents hydroxyl, chlorine or bromine, and

X represents halogen,

with the exception of 4-trifluoromethyl-tetrafluorobenzyl alcohol or its reactive derivatives.

4. Process for the preparation of the new compounds of the formula (IIIa), characterised in that

(a) to prepare compounds of the formula (IIIa) in which Z represents hydroxyl, trifluoromethylbenzene-carbonyl compounds of the formula IV

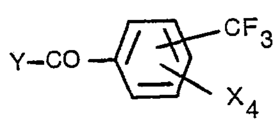

(IV)

in which

X has the abovementioned meanings and

Y represents hydrogen, fluorine or chlorine,

with the exception of a compound of the formula

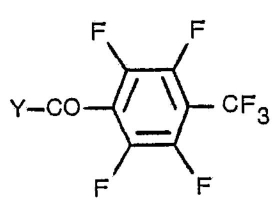

are reacted with sodium boranate, (sodium tetrahydridoborate) or lithium alanate (lithium tetrahydrido-aluminate), if appropriate in the presence of a diluent or

(b) to prepare compounds of the formula IIIa, in which Z represents chlorine or bromine, trifluoromethyltoluene derivatives of the formula V

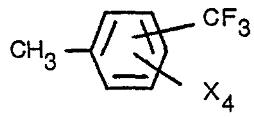

(V)

in which

X has the abovementioned meanings,

with the exception of the compound 4-trifluoromethyltetrafluorotoluene, are reacted with chlorine or bromine, if appropriate in the presence of a diluent and if appropriate in the presence of a catalyst.

5. Agents for combating pests, characterised in that they contain at least one trifluoromethylbenzyl ester of the formula I.

6. Use of trifluoromethylbenzyl esters of the formula (I) for combating pests.

7. Process for combating pests, characterised in that trifluoromethylbenzyl esters of the formula (I) are allowed to act on pests and/or their environment.

8. Process for the preparation of agents for combating pests, characterised in that trifluoromethylbenzyl esters of the formula (I) are mixed with extenders and/or surface-active agents.

**Revendications**

1. Ester trifluorométhylbenzylique de formule I

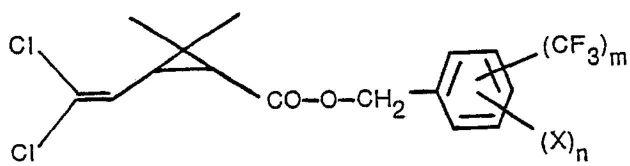

(I)

dans laquelle

X est un halogène,

m a la valeur 1, 2 ou 3 et

n a la valeur 0, 1, 2, 3 ou 4

sous réserve que la somme de m et n se situe entre 2 et 5, à l'exception de l'ester 4-trifluorométhyltétrafluorobenzylique.

2. Procédé de production des composés nouveaux de formule (I), caractérisé en ce qu'on fait réagir l'acide carboxylique de formule II

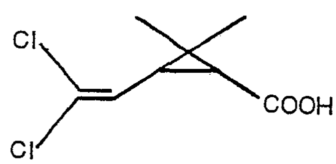

(II)

ou ses dérivés réactifs avec des alcools trifluorométhylbenzyliques de formule III

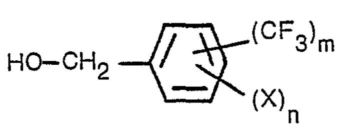

(III)

dans laquelle

X, m et m ont les définitions données ci-dessus, à l'exception de l'alcool 4-trifluorométhyltétrafluorobenzylique, ou avec leurs dérivés réactifs, le cas échéant en présence d'accepteurs d'acides et en la présence éventuelle de diluants.

3. Alcools trifluorométhylbenzyliques et leurs dérives réactifs de formule IIIa

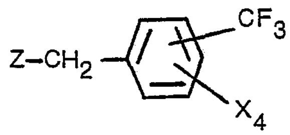

(III–a)

dans laquelle

Z est un groupe hydroxy, le chlore ou le brome,

X est un halogène,

à l'exception de l'alcool 4-trifluorométhyltétrafluorobenzylique ou de ses dérivés réactifs.

4. Procédé de production des composés nouveaux de formule (IIIa), caractérisé en ce que

(a) pour l'obtention de composés de formule (IIIa), dans laquelle Z est un groupe hydroxy, on fait réagir des composés trifluorométhylbenzènecarbonyliques de formule IV

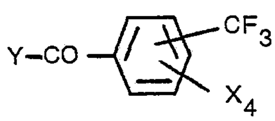

(IV)

dans laquelle

X a les définitions indiquées ci-dessus et

Y représente l'hydrogène, le fluor ou le chlore,

excepté un composé de formule

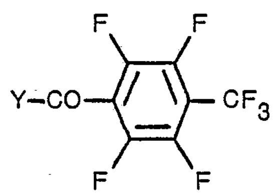

avec le boranate de sodium (tétrahydridoborate de sodium) ou l'alanate de lithium (tétrahydrido-aluminate de lithium) le cas échéant en présence d'un diluant, ou bien

(b) pour l'obtention de composés de formule IIIa, dans laquelle Z représente le chlore ou le brome, on fait réagir des dérivés trifluorométhyltoluéniques de formule V

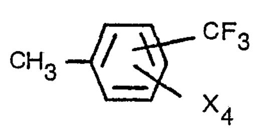

(V)

dans laquelle

X a les définitions indiquées ci-dessus, excepté le 4-trifluorométhyltétrafluorotoluène,

avec du chlore ou du brome, éventuellement en présence d'un diluant et en présence éventuelle d'un catalyseur.

5. Compositions pesticides, caractérisées par une teneur en au moins un ester trifluorométhylbenzylique de formule I.

6. Utilisation d'esters trifluorométhylbenzyliques de formule (I) pour combattre des parasites.

7. Procédé pour combattre des parasites, caractérisé en ce qu'on fait agir des esters trifluorométhylbenzyliques de formule (I) sur les parasites et/ou sur leur habitat.

8. Procédé de préparation de compositions pesticides, caractérisé en ce qu'on mélange des esters trifluorométhylbenzyliques de formule (I) avec des diluants et/ou des agents tensio-actifs.